# EUROPEAN PATENT APPLICATION

(11) **EP 4 517 759 A1**
(43) Date of publication of application: **05.03.2025**
(21) Application number: 24196852.8
(22) Date of filing: 28.08.2024
(51) Int. Cl.: G16B 20/00, G16B 40/00, G16H 50/20

(54) **MEDICAL DECISION SUPPORT SYSTEM USING PROTEIN AND DNA LANGUAGE MODELS**

(30) Priority: 01.09.2023 US 202318459663
(71) Applicant: Siemens Healthineers AG, 91301 Forchheim (DE)
(72) Inventor: SIEBERT, Matthias, 91080 Marloffstein (DE); SINGH, Vivek, Princeton, NJ, 08540 (US); KAMEN, Ali, Skillman, NJ, 08558 (US)
(74) Representative: Horn Kleimann Waitzhofer Schmid-Dreyer Patent- und Rechtsanwälte PartG mbB

(57) **Abstract**

Based on available genomic data for a patient, both a DNA language model (304) and a protein language model (302) are used to generate (135, 145) scores (200, 202) for medical decision support. These scores (200, 202) are used to predict (150) the health of the patient. For example, decision support is provided by scoring expression of genes and the functionality of encoded proteins and inputting the scoring to a predictive model (306) for predicting (150) health of the patient.

## Description

### BACKGROUND

The present embodiments relate to medical decision support based on genomic data. The genetic makeup of cells contributes to, or even determines, disease etiology. For most common, complex diseases, polygenic inheritance, involving many common genetic variants of small effect, plays a greater role than rare monogenic mutations, resulting in a broad range of individual disease risk. In cancer, the focus has been on identifying driver mutations in oncogenes and tumor suppressor genes that impair the function of encoded proteins and ultimately transform a normal cell into a malignant cell. In both cases, genotyping technologies, such as next-generation sequencing, are increasingly being employed to support clinical decision-making. However, the size of genomic territory in combination with small patient cohorts as well as inherent genetic heterogeneity, i.e., different genotypes resulting in the same phenotype, render it difficult to suitably train clinical decision support systems that consider all relevant genomic information. For instance, effects of non-coding mutations in regulatory sequences, such as promoter and enhancer sequences, which determine the expression of a gene (rather than the functionality of the encoded protein) have not been considered on a large scale.

To comprehensively characterize the genetic component of a disease and, thus, improve diagnosis, prognosis, and prediction, e.g., with respect to normal tissue complications or toxicity resulting from treatments such as radiotherapy, it is indispensable to consider the combined effects of protein-coding and non-coding mutations, including inherited (i.e., germline) and acquired (i.e., somatic) mutations, on both the functionality and the expression of proteins. While the expression of a gene and its encoded protein can also be directly measured using transcriptomic and proteomic approaches, genomics is by far the most clinically established and most often the only available molecular modality in clinical routine.

For common, complex diseases, e.g., coronary artery disease, genome-wide polygenic risk scores (PRS) identify high-risk individuals and, thus, enable enhanced screening or preventive therapies, e.g., using the LDPred algorithm. However, PRSs typically do not consider the effect of variants in the context of genes and gene regulatory sequences, not to mention interactions of encoded proteins. In cancer, the standard approach to developing predictive models based on gene mutations is to preselect a task-specific set of genes/mutations previously identified to be independently associated with the phenotype and restrict training of a basic predictive model to this limited set, e.g., learning a Cox regression model from mutations in 11 genes to predict response to immunotherapy. Combinations of somatic mutations that predict cancer survival and immunotherapy benefit have also been identified using unsupervised Restricted Boltzmann Machines. Genomic alterations, and features inferred therefrom, determined with a more comprehensive sequencing panel targeting up to 468 cancer-associated genes have been used to construct a Random Forest classifier to predict tumor sites of origin. Regulatory sequences, such as promoter and enhances elements, are generally not considered, with few exceptions, e.g., TERT promoter mutations.

In addition, there are methods that integrate genomic data with more functional readouts, such as gene and protein expression data. For instance, neural network models have been trained from multiple 'omics modalities to support clinical decisions. Some of the methods also integrated prior knowledge on direct or indirect protein-protein interactions. However, all these methods require access to data for multiple modalities, including transcriptomics and proteomics, during training as well as in clinical routine.

### SUMMARY

Systems, methods, and instructions on computer readable media are provided for medical decision support. Based on available genomic data for a patient, both a DNA language model and a protein language model are used to generate scores. These scores are used to predict the health of the patient. For example, decision support is provided by scoring expression of genes and the functionality of encoded proteins and inputting the scoring to a predictive model.

In a first aspect, a method is provided for decision support in a medical system. A genomic sequence for a patient is acquired. First scores of protein function are generated by a processor-implemented protein language model based on input derived from the genomic sequence. Second scores of gene expression are generated by a processor-implemented regulatory DNA language model based on input derived from the genomic sequence. The health of the patient is predicted by a machine-learned model in response to input of the first scores and second scores to the machine-learned model. The health as predicted by the machine-learned model is displayed.

In a second aspect, a medical decision support system is provided. A memory is configured to store information for a genomic sequence of a patient, a first language model for expression of genes, a second language model for functionality of encoded proteins, and a neural network for prediction of medical condition or disease. A processor is configured to apply the first language model to at least some of the information, apply the second language model to at least some of the information, and to apply the neural network to outputs of the first and second language models, the neural network configured to output decision support information for the patient from the application of the neural network. A display is configured to display the decision support information or information derived from the decision support information.

In a third aspect, a method is provided for the analysis of genomic sequence data. Genomic sequence data of a patient is obtained. Protein-coding gene sequences contained in the genomic sequence data are translated into encoded protein sequences. A protein language model is applied to the encoded protein sequences. The applying results in first metric scores that assess functionality of corresponding proteins. DNA sequences are extracted from the genomic sequence data that exert a regulatory effect on expression of the protein-coding genes. A regulatory DNA language model is applied to the extracted DNA sequences. The applying results in second metric scores that assess the expression of the corresponding protein-coding genes. A diagnostic, prognostic, or predictive output is derived with respect to a disease or medical condition by a machine-trained predictive model in response to input of the first and second metric scores to the machine-trained predictive model. The diagnostic, prognostic, or predictive output is displayed.

Any one or more of the aspects or concepts described above or in the illustrative embodiments below may be used alone or in combination. The aspects or concepts described for one embodiment or aspect may be used in other embodiments or aspects. The aspects or concepts described for a method or system may be used in others of a system, method, or non-transitory computer readable storage medium.

These and other aspects, features, embodiments, and advantages will become apparent from the following detailed description of preferred embodiments, which is to be read in connection with the accompanying drawings. The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale, emphasis instead being placed upon illustrating the principles of the embodiments. Moreover, in the figures, like reference numerals designate corresponding parts throughout the different views.
Figure 1 is a flow chart diagram of one embodiment of a method for medical decision support using language models and genomic data;
Figure 2 illustrates an example graph neural network for prediction from language model scores; and
Figure 3 is a block diagram of one embodiment of a system for medical decision support.

### DETAILED DESCRIPTION OF EMBODIMENTS

For decision support based on genomics, various features may be useful. For example, exclusively depending on genomic sequence data as input enables clinical applicability. As another example, variants in all genomic sequences that together determine the expression of genes and the functionality of encoded proteins are considered. As yet another example, prior knowledge of interactions between proteins is considered to enable robust model training on small patient cohorts. A generic system using one or more of these features has the capability to be adjustable to a multitude of diseases and clinically relevant downstream tasks to improve patient management, such as prognosis or prediction of treatment response.

The features may be provided by using molecular language models. The combination of estimates of both the expression of genes and the functionality of encoded proteins are input to a predictive model. As a result, improved clinical decision support can be provided with respect to a disease or medical condition based solely on genomic sequence data, based on gene expression and protein functionality predicted therefrom, and providing protein-protein interactions.

Molecular sequences, such as protein and DNA sequences, may be scored by transformer architectures configured as language models. For instance, protein language models are trained on large databases of protein sequences, e.g., UniRef90, to capture the functional effects of protein sequence variation, either with or without dependence on multiple protein sequence alignments and supervision from experimental measurements of function. Similarly, DNA regulatory sequences, including distal enhancers, repressors, and/or insulators, which can influence gene expression from far greater than 20 kb away from transcription start sites, the limit of convolutional network-based algorithms for predicting gene expression, can be effectively integrated by the increased receptive field of transformer layers of up to 100 kb in a DNA language model.

Clinical decision making is improved for subjects with a disease or medical condition based (e.g., solely or in part) on genomic sequence data. To consider variants in genomic sequences that together determine the expression and functionality of protein-encoded genes, multiple language models are applied to respective genomic sequences. A protein language model determines metric scores that correspond to the extent each protein is functional based on its protein sequence. A regulatory DNA language model determines metric scores that correspond to the extent each gene is expressed based on its regulatory sequences. The metric scores are used as input to a predictive model, e.g., a neural network, that has been trained or, alternatively, is trained, with respect to a diagnostic, prognostic, or predictive conclusion in the context of a disease or medical condition.

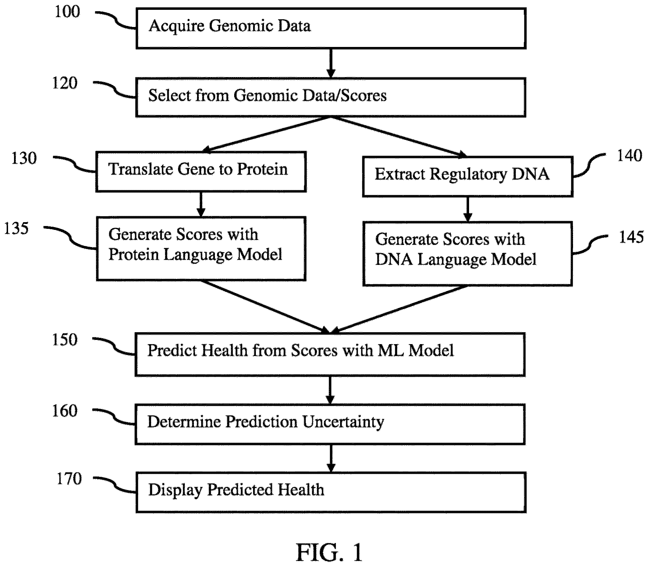

Figure 1 is a flow chart diagram of one embodiment of a method for decision support in a medical system. The method includes analysis of genomic sequence data. Multiple language models score from genomic related data, such as scoring protein function, gene expression (including RNA and/or protein expression), RNA function, protein interaction, or other molecular characteristics. The scores are used to predict health of a patient, such as disease, medical condition, prognosis, therapy response, and/or diagnosis.

The method is performed in the order shown (e.g., top to bottom or numerical), but other orders may be used. For example, act pairs 130, 135 and 140, 145 are performed in any order. As another example, act 120 is performed after or both before and after acts 135 and/or 145.

Additional, different, or fewer acts may be provided. For example, acts 120, 140, 160, and/or 170 are not performed. As another example, acts for extracting different gene sequences are provided, such as functional RNA-coding genes.

The method is performed by a medical system, a workstation, a server, or a computer. A DNA sequencer or other genotyping instrument may be used to acquire genomic sequence data. The processor performs various acts to predict the health from the genomic sequence data. A display displays the prediction. Other devices, circuits, or equipment may be used.

In act 100, a processor acquires a genomic sequence for a patient. Genomic sequence data of a patient is acquired by a sequencer or other genotyping instrument from the patient. The genomic sequence data may be from a tumor sample (i.e., the nucleic acids may be extracted from a tumor of a subject, tissue, urine, blood, semen, liquor, plasma, serum, umbilical cord, or other sample). Liquid biopsy samples derived from blood, urine or other body fluids may be used. Alternatively, the genomic sequence data is acquired from a memory or transferred over a computer network, such as from a previously performed assay of a sample.

Only genomic sequence data is used for prediction. In other approaches, genomic sequence data and other data, such as from an electronic medical record for the patient, are acquired and used for prediction.

The genomic sequence data includes targeted gene panel sequencing, such genetic (i.e., DNA) information for a selected group of genes (panel), whole-exome sequencing, whole-genome sequencing, whole-genome genotyping microarray, and/or imputation of unobserved genotypes. The nucleotide sequence of either individual nucleic acid molecules or expanded clones for individual nucleic acid molecules are obtained. The panel and resulting genomic sequence data may be specific to protein-coding genes, corresponding regulatory DNA sequences, and/or a disease or medical condition of interest related genes. For imputation, unobserved genotypes (e.g., genotypes that have not been directly assayed) are inferred from a genotype reference sequence or population of genotype sequences.

Genomic sequence data is sequence data obtained by any technique suitable to provide sequence data of an organism's genome. The genomic sequence may include any genomic sequence segment of interest for the determination of the presence of a mutation. Coding sequences, and/or non-coding sections (e.g., regulatory sequences, regulatory active intron sequences, etc.) may be acquired. The genomic sequence data may include primarily exonic sequences or exonic sequences. The genomic sequence data may further be limited to certain chromosomes, chromosomal regions, gene clusters, gene families, or genes.

In act 120, the processor selects from the genomic sequence data. The entire sequence may be used, such as a whole genome sequence for the patient or all the genetic sequences provided from a panel. In act 120, only part of the available genomic sequence data is used. For example, any protein-coding genes, corresponding regulatory DNA sequences, and/or genes related to disease or medical condition, are selected.

The genomic sequence data to be used as input to one or more language models is selected. Some of the available genomic sequence data is not selected. Different genomic sequence data may be selected for different language models (e.g., different selections for a regulatory DNA language model and the protein language model).

The selection occurs in the genomic sequence data. The selected data may be transcribed to functional RNA and/or translated to protein sequences. For functional RNAs or proteins, the selection may occur after transcription/translation from DNA to RNA sequence or protein sequence.

Specific genes known for their implication in disease etiology may be selected. For example, in the context of cancer genes that are annotated in the COSMIC Cancer Gene Census and carry one or more non-synonymous mutations may be selected. Preferably, e.g., in the context of cancer, the genes encoding TP53, KRAS, KEAP1, STK11, EGFR, NF1, BRAF or STED2 may be preselected, and corresponding genomic sequence data be obtained. Further specific examples of genes involved in different classes of diseases would be known to the skilled person or can be derived from suitable databases.

In an alternative or additional approach, the selection of act 120 is of the scores output from acts 135 and/or 145. The outputs of the language models are selected for input to the prediction of act 150. Less than all the scores and corresponding genes, functional RNAs, and/or proteins are selected. For example, a threshold score is applied. A preselection of genomic sequence data with respect to protein-coding genes is obtained by applying a minimum threshold to the metric scores obtained from the DNA language model in act 145.

The genomic sequence data or data derived therefrom (e.g., protein sequences) is selected for use by two or more language models. The sequence data (e.g., DNA sequence and/or protein sequence) is coded (e.g., combinations of four bases ACGT for DNA, four bases ACGU for RNA, and twenty amino acids for protein). The coding is treated as a language. The language models predict characteristics from the sequence data, such as gene expression and protein function or interaction. To consider variants in genomic sequences that together determine the expression and functionality of protein-encoded genes, both a protein language model for scoring functionality of each protein and a regulatory DNA language model for scoring extent a gene is expressed are used. Other language models for scoring other characteristics may be used as well or instead. The genomic sequence data (or information derived therefrom) is used to score by language models, which scores may then be used to predict health.

In act 130, the processor translates protein-coding gene sequences contained in the genomic sequence data into encoded protein sequences. Any translation program may be used, such as based on a database relating gene sequences to protein sequences. Translation *in silico* code is provided. The obtained genomic sequence data is converted to the protein sequence. The conversion is performed from the nucleotide sequence to the encoded amino acid sequence of the protein.

The genomic and/or protein sequence data may be modified or processed. For example, the sequence data is transformed into a new format, analyzed with respect to a feature of interest, or combined in any suitable way.

In act 135, the processor generates one or more scores for protein function. One or more scores may be generated for each of one or more proteins or protein combinations. In one approach, one score is provided for each of multiple proteins or protein-protein interactions.

The score is a metric for the characteristic or characteristics of interest. For example, a score over a range is provided. In proteins, this range may be [0, 1] where 1 represents no mutation (e.g., fully functional) and 0 represents highest impact mutation(s) (e.g., not functional) with values in-between representing different levels of mutation or function. Other ranges and/or representations may be used.

The scores are generated by a processor-implemented protein language model in response to input derived from the genomic sequence. The translated genes are input as protein sequences to the protein language model, which generates the output scores in response. The protein language model determines metric scores that correspond to the extent each protein is functional based on its protein sequence. By input of different, particular, and/or all protein sequences for the patient, scores for protein function are generated. The protein language model is applied to the protein sequences, thereby obtaining metric scores that assess the functionality of the corresponding proteins.

Any protein language model may be used. The protein language model may be a machine-learned model, such as a transformer neural network previously trained to score based on input of protein sequence. In one embodiment, the protein language model is an Evolution Scale Model (ESM), such as ESM-b, ESM-v1, or ESM-2 model. Other ESM or other protein language models may be used.

The transformer architecture may be trained to operate on molecular sequences, such as protein and DNA sequences. For instance, protein language models have been trained on large databases of protein sequences, e.g., UniRef90, to capture the functional effects of protein sequence variation, either with or without dependence on multiple protein sequence alignments and supervision from experimental measurements of function.

In act 140, the processor extracts DNA sequences from the genomic sequence data. The DNA sequences that exert a regulatory effect on expression of the protein-coding genes are extracted. Other sequences may be extracted instead or as well. Coding and/or non-coding (e.g., promotor) sequences may be extracted.

The extraction may be based on gene expressions of interest. For example, genes known to correlate with health in general, therapy response, prognosis, diagnosis, medical condition, and/or disease are identified from a database or study. The DNA sequence within a range of these genes in the genomic sequence is extracted as having a regulatory effect. For example, the scores are to be generated with inputs derived from the genomic sequence being within 20, 50, 100, or more kb around the gene, such as around transcription start sites of the gene sequence. DNA regulatory sequences, including distal enhancers, repressors, and insulators, which may influence gene expression from far greater than 20 kb away from transcription start sites, the limit of convolutional network-based algorithms for predicting gene expression, are extracted and can be effectively integrated by the increased receptive field of transformer layers of up to 100 kb or more. Less range may be provided.

The transcription start sites are derived from a database or study. In one approach, the transcription start sites are looked-up from an atlas, such as the FANTOM5 atlas. Other atlases or sources may be used.

In act 145, the processor generates scores of gene expression. One or more scores may be generated for each of one or more genes or gene combinations. In one approach, one score is provided for each of multiple regulatory DNA sequences.

The score is a metric for the characteristic or characteristics of interest. For example, a score over a range is provided. In genes, the score may be in the positive range of values where 0 represents no expression and values greater than 0 representing different extents of expression. Other ranges and/or representations may be used.

The scores are generated by a processor-implemented regulatory DNA language model based on input derived from the genomic sequence. The extracted regulatory DNA sequences for the genes of interest are input. For example, the input is the 100kb or other range of DNA sequence around the transcription start site of each protein-coding gene of the genomic sequence. A score is generated for each DNA sequence corresponding to each protein-coding gene. Other genes or DNA may be input as well (e.g., genes of interest) or instead of regulatory DNA sequences. A regulatory DNA language model determines metric scores that correspond to the extent each gene is expressed based on its regulatory sequences. The regulatory DNA language model is applied to the regulatory sequences, thereby obtaining metric scores that assess the expression of the corresponding protein-coding genes.

Any regulatory DNA language model may be used. For example, a machine-learned language model is used, such as a transformer neural network-based model. The model is trained to generate a metric score in response to input of the extracted regulatory DNA sequence. In one embodiment, the regulatory DNA language model is an Enformer model. Other models may be used.

Other language models may be used to generate scores. The genomic sequence data of the patient (e.g., extraction) or a derivation (e.g., translation) thereof is input to the other language models to generate scores for sequences of interest. For example, scores are generated based on functional RNA-coding genes as well as the protein-coded genes. Metric scores for functional RNA-coding genes, such as long non-coding RNAs (IncRNAs), are incorporated. Other characteristics, DNA, RNA, or proteins may be used for scoring.

In act 150, the processor predicts health of the patient. The health of the patient may be a diagnostic, prognostic, or predictive output with respect to a disease or medical condition. The prediction is provided by a predictive model trained with respect to a diagnostic, prognostic, or predictive conclusion in the context of a disease or medical condition corresponding to genomic sequence data from a cohort of subjects affected by said disease or medical condition.

The health may be reflected by a classification, such as indicating a type, level, or magnitude of the medical condition or disease. The health may be reflected as a value for a clinical decision scale. A metric score is estimated where the score represents the level of disease or medical condition. For example, the scale is a range (e.g., [0, 1]) where one extreme of the range maps to an extreme of the prediction and the other extreme of the range maps to the other extreme of the prediction (e.g., 1 is 100% probability of response to treatment and 0 is 0% probability of response to treatment). Any range or clinical decision scale may be used.

In another approach, the health is a disease sub-type classification (e.g., genetic type of cancer), prognostic trend assessment (e.g., 5-year survivability), and/or treatment response prediction. As one example, the predictive model is trained to predict the risk of toxicity after radiotherapy treatment of lung cancer patients based on genomic and outcome data generated from the REQUITE cohort. In this data set, genome-wide genotyping data have been imputed from Illumina Infinium OncoArray-500K BeadChip data, while radiotherapy toxicity has been assessed using the Common Terminology Criteria for Adverse Events (CTCAE).

The disease or condition may be cancer or a genetic disorder, such as a hereditary genetic disease. Examples of diseases or medical conditions include cancer such as stomach, colon, rectal, liver, pancreatic, lung, breast, cervix uteri, corpus uteri, ovary, prostate, testis, bladder, renal, brain/CNS, head and neck, throat, Hodgkin's disease, non-Hodgkin's lymphoma, multiple myeloma, leukemia, melanoma skin cancer, non-melanoma skin cancer, acute lymphocytic leukemia, acute myelogenous leukemia, Ewing's sarcoma, small cell lung cancer, choriocarcinoma, rhabdomyosarcoma, Wilms' tumor, neuroblastoma, hairy cell leukemia, mouth/pharynx, oesophagus, larynx, kidney cancer, lymphoma, or any subtype thereof or neurologic diseases such as Alzheimer's disease, multiple sclerosis, Amyotrophic Lateral Sclerosis (ALS), or ataxia, or cardiovascular diseases such as coronary heart diseases; or metabolic diseases such as diabetes, metabolic syndrome, iron metabolism disorders, lipid metabolism disorders, disorders of calcium metabolism etc. Further, the disease or condition may be a predisposition for a disease reflected by the presence of a combination of gene variants or gene modifications.

The prediction is in response to input to the predictive model. The scores generated by the language models in acts 135, 145, and/or any other language model scores are input. The extracted and/or translated sequences of interest are used to generate the scores, which scores are input to the predictive model to predict the health of the patient. A diagnostic, prognostic, or predictive output with respect to a disease or medical condition is predicted by the predictive model in response to input of the metric scores to the predictive model.

In one approach, the scores alone, as provided by the language models using only the genomic sequence data, are used to make the prediction. In other approaches, additional information from the genomic data alone is used in the prediction, such as inputting extracted or translated sequences or genomic information without an intervening language model in addition to the scores from the language model.

In yet another approach, additional inputs from sources other than the genomic sequence data is used. For example, other inputs to the prediction model for prediction include age, sex, race, characteristics of disease phenotypes (e.g., histologic characteristics), stage of development of a disease, detectable molecular changes (e.g., on the level of the transcriptome, proteome, metabolome, or lipidome), and/or biomarkers (e.g., derived from medical images, pathology images, and/or laboratory tests). Different or additional inputs may be used.

The prediction model is a machine-learned model. The metric scores are used as input to a predictive model, e.g., a neural network, that has been machine trained with respect to a diagnostic, prognostic, or predictive conclusion in the context of a disease or medical condition. Any machine-learned model may be used. For example, a support vector machine, clustering, neural network, or another model is provided for generating an estimate of the health based on the input.

Various architectures may be used. The architecture defines the arrangement of layers, units, or other machine learning parts. For a neural network, the architecture may include one or more convolutional layers where the deep learning trains the kernel or kernels for filtering (convolution). Fully connected layers, max pooling layers, batch normalization, and/or other layers may be included. The network is a deep architecture, which may include a convolutional neural network (CNN) or a deep belief net (DBN). Other deep networks may be used.

The neural network is defined as a plurality of sequential feature units or layers. Within a unit or layer, any number of nodes is provided. The nodes of each layer or unit may connect with all or only a sub-set of nodes of a previous or subsequent layer or unit. The features of the nodes are learned by the machine using any building blocks. For example, auto-encoder (AE) or restricted Boltzmann machine (RBM) approaches are used. In other embodiments, at least one unit is a convolution with ReLU activation or is a batch normalization with a ReLU activation followed by a convolution layer (BN+LeakyRU+convolution). Max pooling, up sampling, down sampling, and/or softmax layers or units may be used. Different units may be of the same or different type.

In training, the values of the learnable parameters (features) are learned. Using objective criterion or ground truth (e.g., unsupervised or supervised), an optimization is performed with many samples of paired inputs and outputs. Any optimization, such as ADAM, and corresponding loss may be used, such as L1 norm, L2 norm, or cross-entropy.

In one embodiment, the machine-learned predictive model is a graph neural network. The architecture of the neural network is formed as a graph representing, at least in part, protein-protein interactions. A protein-protein interaction network incorporates prior knowledge on protein-protein interactions. This prior knowledge is incorporated into the predictive model in the form of a graph neural network to consider proteins in the context of biological complexes, functions, and/or pathways. For example, edges between nodes for different proteins are weights representing a likelihood of physical association of connected protein pairs. The weights correspond to a continuous metric of physical association for each connected protein pair. The weights may be fixed where the relationship is known. Alternatively, the weights are initialized using the known relationship but then learnable or are learnable. The existence of the edge and/or value of the weight incorporate the prior knowledge.

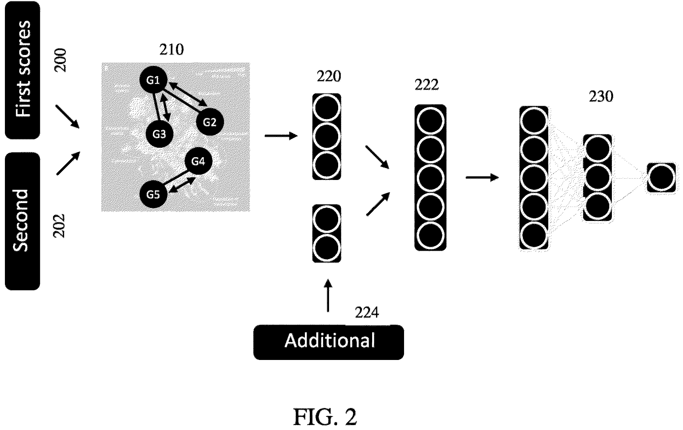

FIG. 2 illustrates of an example graph neural network and further network architecture for predicting from the scores 200, 202. The scores 200, 202 from the language models are input to the graph neural network 210. The graph neural network 210 represents protein-protein interactions where each node G corresponds to a gene/encoded protein and is assigned a tuple of the corresponding scores 200, 202. Each undirected edge corresponds to an interaction between two proteins with arrows illustrating the messages passed between proteins G1-5 as graph convolutional layers are applied. The readout of the graph neural network 210 produces a graph embedding 220. Additional information 224 (e.g., age, race or sex) may be input as an encoding and concatenated with graph embedding 220. The resulting layer 222 is provided to a prediction head, such as a multilayer perceptron 230, producing a prediction label. Other arrangements with additional, different, or fewer layers may be used.

The graph neural network is based on a neighborhood aggregation or message passing scheme, where the representation vector of a node is computed recursively aggregating and transforming representation vectors of its neighboring nodes, i.e., each node aggregates feature vectors of its neighbors to compute its new feature vector. Generally, after a certain number of iterations of aggregation, a node is represented by its transformed feature vector, which captures information within the nodes' neighborhood. The representation of an entire graph can then, for example, be obtained through pooling, e.g., by summing the representation vectors of all nodes in the graph.

The graph neural network is accordingly to be understood as an optimizable transformation on all attributes of the graph, e.g., nodes, edges and global context which preserve graph symmetries. Typically, the graph neural network provides a model for one protein-protein interaction network in the form of one graph.

An adjacency matrix as a square matrix is used to represent a finite graph. The elements of the matrix indicate whether pairs of nodes are adjacent or not in the graph. In addition, or alternatively, an adjacency list may be generated, wherein said list describes the connectivity of edges between nodes as tuples.

The interaction or relationships forming the graph neural network may be determined from databases or studies. Examples for databases include STRING and IntAct. Examples for studies include the integrated association stringency (IAS) score network or the OncoPPi network.

In one embodiment, the graph neural network is a graph convolutional network, a graph isomorphism network, or a graph attention network. A graph convolutional network shares filter parameters over all locations in the graph. In another embodiment, the graph neural network is a graph isomorphism network. A graph isomorphism network implements an aggregation scheme that represents universal functions over a node and the multiset of its neighbors. The graph isomorphism network typically trains a separate multilayer perceptron on each component of the graph, yielding graph isomorphism network layers. Accordingly, for each node vector, the multi-layer perceptron is applied and yields a node feature vector. In yet another embodiment, the graph neural network is a graph attention network. The graph attention network operates on graph-structured data. The attention mechanism deals with variable sized inputs, focusing on the most relevant parts of the input to make decisions. The graph attention network computes hidden representations of each node in the graph by attending over its neighbors, following a self-attention strategy.

The trained graph neural network is or has been trained based on genomic sequence data from a cohort of subjects affected by a disease or medical condition. Accordingly, genomic data is associated with a certain cohort or group of persons known to be affected by a disease or medical condition. The group may be affected by the same disease or condition, or by a family or group of similar diseases or conditions. In addition, genomic data of one or more healthy subjects may be additionally input and used for reference and comparison purposes.

The disease or medical condition is reflected by the subject's genomic sequence, e.g., in the form of a detectable mutation. Such a mutation may be an insert, a deletion, or a substitution of one or more nucleotides. Such mutations may, in further embodiments, lead to changes in the primary amino acid sequence of a protein. After training, the machine-learned predictive model outputs the health of a patient with respect to the disease or medical condition in response to input of the scores from the language models with or without other inputs. The trained predictive model thus produces an output for input scores from genomic sequence data in the context of the learned task, providing a diagnostic, prognostic and/or predictive conclusion with respect to the disease or medical condition originally associated with the training data of the cohort of diseased subjects in the context of the genomic sequence data of the target subject. In other words, the machine-learned predictive model produces an output which links a diagnosis, prognosis and/or therapeutic instruction to the genomic sequence data for the patient based on the language model scoring.

In act 160 of Figure 1, the processor determines an uncertainty of the health as predicted by the machine-learned model. The machine-learned model may output the health (e.g., metric score for clinical decision) as a probability. The probability reflects the uncertainty. As another example, similar patients are identified. One or more patients with the closest combination of metric scores from the language model and/or genomic or patient characteristics may be identified. The outcomes for those patients may be analyzed for variance to indicate an uncertainty in the prediction by the machine-learned prediction model. In another example, the output prediction is mapped to a specific class (e.g., Yes or No for 5-year survivability). A majority vote based on different predictive models for different genes and/or proteins is used to determine the uncertainty. Using bootstrapping in training, different predictive models may be trained from the same training data. Outputs for the different predictive models may be used to determine the uncertainty.

In one approach, the uncertainty is a statistical factor reflecting the accuracy of the prediction by considering the similarity between the current molecular situation of a target subject vis-à-vis the molecular situation of the cohort of subjects yielding the training data set. The statistical factor may, for example, be derived from a measurement of the similarity between the latent representation of the target subject's genomic data and the latent representations of the patient cohorts' genomic data. In a further embodiment, the statistical factor may be derived from the measurement of patient similarity, e.g., with respect to age, sex, race, health records etc. A measurement of similarity may, for example, be performed with a taxicab or Manhattan geometry, wherein the distance between two points is measured as sum of absolute differences of Cartesian coordinates. For example, in case a target subject's genomic sequence data yields a mutational pattern, which is reflected by mutational data derivable from subjects of the training cohort, more preferably by data of subjects which have been treated successfully, the likelihood of a positive therapeutic response of the target subject is high. In consequence, the uncertainty would be low.

In act 170, a display displays the health as predicted by the machine-learned model. The processor generates an image, which image is displayed on a display. The image indicates the diagnostic, prognostic, or predictive output with respect to the disease and/or medical condition. The image may be output to a display, into a patient medical record, and/or to a report.

The health may be an annotation, alphanumeric text, graph, or chart. For example, the survival is displayed as a graph of survival probability as a function of time. As another example, stratification results are displayed. The prediction score is displayed for the physicians. The output may, for example, be provided in a report or as an alert.

Other information may be displayed with the predicted health, such as input information (e.g., genomic sequence data) strongly linked with the prediction and/or the uncertainty. More than one prediction may be output, such as (1) therapy outcome predictions for different therapies or (2) diagnosis and prognosis.

The output health may be presented with or on (e.g., overlay or annotation) an image of the patient. For example, an image of a slice through a tumor region is displayed. The output health is provided with the image of the anatomical structure of interest, such as the lung or gross tumor volume, on a display screen.

The display may include information from one or more similar patients. The language model scores and/or other information may be used to find other or past patients with a similar arrangement of scores.

In one embodiment, the metric score output by the predictive model on the clinical decision scale is output. The metric score is a rank along a graduation scheme. This graduation scheme is provided as metric classification embedded within a clinical or therapeutic decision scale. For example, the severity of a disease may be classified on a scale from 1 to 10, e.g., 1 being very mildly severe, whereas 10 is strongly severe. This diagnosis may subsequently lead to a corresponding therapeutic decision. Alternatively, the output may be a value for the likelihood of, for example, a 6-month survival (e.g., after mutations causing cancer were detected). In a further example, the output may be a prediction of the time until recurrence of the disease. Likewise, the predictive output may be provided as metric score, e.g., a certain dosage may be provided in a metric scale from 1 to 10 times a normalized dose 1, or a radiation therapy may be suggested with a metric graduation from strength 1 to 10 based on a normalized standard strength 5.

Based on the displayed health, the physician assists the patient. For example, the diagnosis and/or prognosis may be confirmed and communicated. As another example, possible outcomes of one or more treatments may be analyzed, and a treatment selected for the patient. The physician uses information about the patient and the output health to select a therapy or to not select a therapy. The stratification, survival, and/or other outcome are used to select the treatment for the patient.

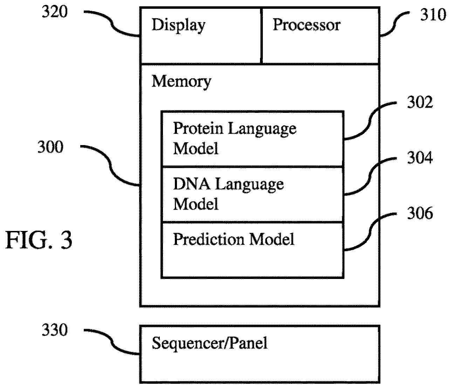

Figure 3 shows a medical decision support system. The system generates an estimate of health of a patient. Genomic sequence data and language models 302, 304 are used to predict the health of a patient by a prediction model 306.

The medical decision support system includes the display 320, memory 300, and processor 310. A sequencer 330 may be included for acquiring the genomic sequence data of the patient. The display 320, processor 310, and memory 300 may be part of a computer, server, workstation, or other system for medical diagnosis, prognosis, and/or treatment of a patient.

Additional, different, or fewer components may be provided. For example, a computer network is included for remote prediction based on locally captured sequencing data. As another example, a user input device (e.g., keyboard, buttons, sliders, dials, trackball, mouse, or other device) is provided for user interaction with the prediction. In yet another example, the sequencer 330 is not provided, such as where the memory 300 stores the genomic sequence data or data derived therefrom (e.g., extracted regulatory sequences and translated protein sequences).

The memory 300 is configured by formatting and/or the processor 310 to store information for a genomic sequence of a patient. The memory 300 also or alternatively stores the protein language model 302, DNA language model 304, and/or prediction model 306 (e.g., a neural network for prediction of a medical condition or disease). In one embodiment, the protein language model 302 is for functionality of encoded proteins, and the DNA language model 304 is for expression of genes. For example, the DNA language model is a machine-learned transformer as a regulatory DNA language model. This regulatory DNA language model operates on DNA 20kb, 50kb, 100kb, or more around transcription start sites in the genomic sequence of particular genes of interest for the medical condition or disease. As another example, the protein language model 302 is a machine-learned transformer as a model for determining functionality of proteins based on the protein sequence.

The prediction model 306 as stored in the memory 300 is a machine-learned model, such as a neural network, graph neural network, or another architecture or type, for predicting health from, at least in part, scores output by the language models 302, 304, .... As a graph neural network, at least part of the network incorporates prior knowledge based on protein-protein interactions.

The memory 300 is an external storage device, RAM, ROM, database, and/or a local memory (e.g., solid state drive or hard drive). The same or different non-transitory computer readable media may be used for the instructions and other data. The memory 300 may be implemented using a database management system (DBMS) and residing on a memory, such as a hard disk, RAM, or removable media. Alternatively, the memory 300 is internal to the processor 310 (e.g., cache). The memory 300 is formed from one device or a collection of devices, such as different memories storing different types of data.

The instructions for implementing the training or application process (i.e., prediction for a patient), the methods, and/or the techniques discussed herein by the processor 310 are provided on non-transitory computer-readable storage media or memories, such as a cache, buffer, RAM, removable media, hard drive or other computer readable storage media (e.g., the memory 300). Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination.

In one embodiment, the instructions are stored on a removable media device for reading by local or remote systems. In other embodiments, the instructions are stored in a remote location for transfer through a computer network. In yet other embodiments, the instructions are stored within a given computer, CPU, GPU, or system. Because some of the constituent system components and method steps depicted in the accompanying figures may be implemented in software, the actual connections between the system components (or the process steps) may differ depending upon the manner in which the present embodiments are programmed.

The processor 310 is a control processor, general processor, digital signal processor, graphics processing unit, application specific integrated circuit, field programmable gate array, artificial intelligence processor or accelerator, digital circuit, analog circuit, combinations thereof, or other now known or later developed device for processing language models, genomic sequence data, and machine-learned prediction models. The processor 310 is a single device, a plurality of devices, or a network. For more than one device, parallel or sequential division of processing may be used. Different devices making up the processor 310 may perform different functions. The processor 310 operates pursuant to stored instructions, hardware, and/or firmware to perform various acts described herein.

In one embodiment, the processor 310 is configured to train one or more machine learning networks, such as the transformers forming the language models 302, 304 and/or neural network forming the prediction model 306. Based on a user provided or other source of the network architecture and training data, the processor 310 learns features or values of learnable parameters to train the network.

Alternatively, or additionally, the processor 310 is configured to apply one or more machine-learned models. For example, the processor 310 is configured to translate genomic sequence data to protein sequences and apply the protein language model 302 to at least some of the translated information. As another example, the processor 310 is configured to extract DNA or gene sequences (e.g., regulatory DNA) from the genomic sequence data and apply the DNA language model 304 to at least some of the extracted information. In yet another example, the processor 310 is configured to apply the prediction model 306 (e.g., neural network) to outputs of the language models 302, 304. The prediction model 306 is configured by previous training to output decision support information (e.g., health) for the patient from the application of the prediction model 306. For example, the health (e.g., diagnosis, prognosis, and/or therapy response) for the patient is output.

The processor 310 is configured to generate an image. An image showing the predicted information is generated. The information with respect to a disease or medical condition may be displayed with an image of the interior of the patient, such as a computed tomography image, or alone.

The predicted information is displayed for decision support. The image shows the decision support information or information derived from the decision support information (e.g., recommended treatment given predictions of success of different treatments output by the prediction model 306, including an explanation of the results, e.g., providing feature attributions).

The display 320 is a CRT, LCD, projector, plasma, printer, tablet, smart phone or other now known or later developed display device for displaying the decision support information or information derived from the decision support information. The display 320 is configured by loading an image to a display buffer or plane, which image is then displayed on a screen. Other configuration may be provided, such as configuring for display by printing. The display 320 is configured to display the decision support information or information derived from the decision support information.

Listed below are various illustrative embodiments.

Illustrative embodiment 1. A method for decision support in a medical system, the method comprising: acquiring a genomic sequence for a patient; generating first scores of protein function by a processor-implemented protein language model based on input derived from the genomic sequence; generating second scores of gene expression by a processor-implemented regulatory DNA language model based on input derived from the genomic sequence; predicting health of the patient by a machine-learned model in response to input of the first scores and second scores to the machine-learned model; and displaying the health as predicted by the machine-learned model.

Illustrative embodiment 2. The method of illustrative embodiment 1 wherein predicting comprises predicting the health as a value in a clinical decision scale for a disease or medical condition.

Illustrative embodiment 3. The method of any illustrative embodiment 1-2 further comprising determining an uncertainty of the health as predicted by the machine-learned model.

Illustrative embodiment 4. The method of any illustrative embodiment 1-3 wherein generating the first scores comprises generating the first scores by the processor-implemented protein language model comprising an Evolution Scale Model (ESM), and wherein generating the second scores comprises generating the second scores by the processor-implemented regulatory DNA language model comprising an Enformer model.

Illustrative embodiment 5. The method of any illustrative embodiment 1-4 wherein generating the second scores comprises generating the second scores with the input derived from the genomic sequence being 100 kb or more around transcription start sites of protein-coding genes of the genomic sequence.

Illustrative embodiment 6. The method of illustrative embodiment 5 further comprising deriving the transcription start sites from an atlas.

Illustrative embodiment 7. The method of any of illustrative embodiments 5-6 wherein generating the second scores further comprises generating based on functional RNA-coding genes as well as the protein-coding genes.

Illustrative embodiment 8. The method of any illustrative embodiment 1-7 wherein predicting comprises predicting where the machine-learned model comprises a neural network.

Illustrative embodiment 9. The method of illustrative embodiment 8 wherein the neural network comprises a graph neural network representing protein-protein interaction network.

Illustrative embodiment 10. The method of illustrative embodiment 9 wherein edges of the graph neural network comprise weights representing a likelihood for a physical association of connected protein pairs.

Illustrative embodiment 11. The method of any illustrative embodiment 1-10 wherein predicting comprises predicting in response to the input where the input further comprises age, sex, race, histologic characteristics, stage of development of a disease, detectable molecular changes, biomarkers derived from medical images, pathology images, and/or laboratory tests.

Illustrative embodiment 12. The method of any illustrative embodiment 1-11 further comprising selecting from the genomic sequence with respect to protein-coding genes, corresponding regulatory DNA sequences, and/or disease or medical condition.

Illustrative embodiment 13. The method of illustrative embodiment 12 wherein the input derived from the genomic sequence for the regulatory DNA language model and/or the protein language model is derived from the selection.

Illustrative embodiment 14. The method of any illustrative embodiment 12-13 wherein selecting comprises selecting based on a threshold of the first or second scores.

Illustrative embodiment 15. The method of any illustrative embodiment 1-14 wherein acquiring comprises acquiring from a gene panel sequencing, whole-exome sequencing, whole-genome sequencing, whole-genome genotyping microarray, and/or imputation of unobserved genotypes.

Illustrative embodiment 16. A medical decision support system comprising: a memory configured to store information for a genomic sequence of a patient, a first language model for expression of genes, a second language model for functionality of encoded proteins, and a neural network for prediction of medical condition or disease; a processor configured to apply the first language model to at least some of the information, apply the second language model to at least some of the information, and to apply the neural network to outputs of the first and second language models, the neural network configured to output decision support information for the patient from the application of the neural network; and a display configured to display the decision support information or information derived from the decision support information.

Illustrative embodiment 17. The medical decision support system of illustrative embodiment 16 wherein the first language model comprises a regulatory DNA language model where the at least some of the information to which the first language model is applied comprises 100 kb or greater around transcription start sites in the genomic sequence.

Illustrative embodiment 18. The medical decision support system of any illustrative embodiment 16-17 wherein the neural network comprises a graph neural network arranged based on protein-protein interactions.

Illustrative embodiment 19. A method for the analysis of genomic sequence data, the method comprising: obtaining genomic sequence data of a patient; translating protein-coding gene sequences contained in the genomic sequence data into encoded protein sequences; applying a protein language model to the encoded protein sequences, the applying resulting in first metric scores that assess functionality of corresponding proteins; extracting DNA sequences from the genomic sequence data that exert a regulatory effect on expression of the protein-coding genes; applying a regulatory DNA language model to the extracted DNA sequences, the applying resulting in second metric scores that assess the expression of the corresponding protein-coding genes; deriving a diagnostic, prognostic, or predictive output with respect to a disease or medical condition by a machine-trained predictive model in response to input of the first and second metric scores to the machine-trained predictive model; and displaying the diagnostic, prognostic, or predictive output.

Illustrative embodiment 20. The method of illustrative embodiment 19 wherein deriving comprises deriving by the machine-trained predictive model comprises a graph neural network where the graph represents protein-protein interactions.

Various improvements described herein may be used together or separately. Although illustrative embodiments of the present invention have been described herein with reference to the accompanying drawings, it is to be understood that the invention is not limited to those precise embodiments, and that various other changes and modifications may be affected therein by one skilled in the art without departing from the scope or spirit of the invention.

## Claims

1. A method for decision support in a medical system, the method comprising:
acquiring (100) a genomic sequence for a patient;
generating (135) first scores (200) of protein function by a processor (310)-implemented protein language model (302) based on input derived from the genomic sequence;
generating (145) second scores (202) of gene expression by a processor (310)-implemented regulatory DNA language model (304) based on input derived from the genomic sequence;
predicting (150) health of the patient by a machine-learned model (306) in response to input of the first scores (200) and second scores (202) to the machine-learned model (306); and
displaying (170) the health as predicted by the machine-learned model (306).

2. The method of claim 1 wherein predicting (150) comprises predicting (150) the health as a value in a clinical decision scale for a disease or medical condition.

3. The method of claim 1 or 2 further comprising determining (160) an uncertainty of the health as predicted by the machine-learned model (306).

4. The method of any one of claims 1-3, wherein generating (135) the first scores (200) comprises generating (135) the first scores (200) by the processor (310)-implemented protein language model (302) comprising an Evolution Scale Model (ESM), and wherein generating (145) the second scores (202) comprises generating (145) the second scores (202) by the processor (310)-implemented regulatory DNA language model (304) comprising an Enformer model.

5. The method of any one of claims 1-4, wherein generating (145) the second scores (202) comprises generating (145) the second scores (202) with the input derived from the genomic sequence being 100 kb or more around transcription start sites of protein-coding genes of the genomic sequence.

6. The method of claim 5 further comprising deriving the transcription start sites from an atlas.

7. The method of claim 5 or 6 wherein generating (145) the second scores (202) further comprises generating based on functional RNA-coding genes as well as the protein-coding genes.

8. The method of any one of claims 1-7, wherein predicting (150) comprises predicting (150) where the machine-learned model (306) comprises a neural network.

9. The method of claim 8 wherein the neural network comprises a graph neural network (210) representing protein-protein interaction network.

10. The method of claim 9 wherein edges of the graph neural network (210) comprise weights representing a likelihood for a physical association of connected protein pairs.

11. The method of any one of claims 1 - 10, wherein predicting (150) comprises predicting (150) in response to the input where the input further comprises age, sex, race, histologic characteristics, stage of development of a disease, detectable molecular changes, biomarkers derived from medical images, pathology images, and/or laboratory tests.

12. The method of any one of claims 1 - 11, further comprising selecting from the genomic sequence with respect to protein-coding genes, corresponding regulatory DNA sequences, and/or disease or medical condition.

13. The method of claim 12 wherein the input derived from the genomic sequence for the regulatory DNA language model (304) and/or the protein language model (302) is derived from the selection.

14. The method of claim 12 or 13 wherein selecting comprises selecting based on a threshold of the first or second scores (202).

15. The method of any one of claims 1 - 14, wherein acquiring (100) comprises acquiring (100) from a gene panel sequencing, whole-exome sequencing, whole-genome sequencing, whole-genome genotyping microarray, and/or imputation of unobserved genotypes.

16. A medical decision support system comprising:
a memory (300) configured to store information for a genomic sequence of a patient, a first language model (304) for predicting expression of genes, a second language model (302) for predicting (150) functionality of encoded proteins, and a neural network (306) for prediction of medical condition or disease;
a processor (310) configured to apply the first language model (304) to at least some of the information, apply the second language model (302) to at least some of the information, and to apply the neural network (306) to outputs of the first and second language models (302, 304), the neural network (306) configured to output decision support information for the patient from the application of the neural network(306); and
a display (320) configured to display the decision support information or information derived from the decision support information.

17. The medical decision support system of claim 16 wherein the first language model (304) comprises a regulatory DNA language model (304) where the at least some of the information to which the first language model (304) is applied comprises 100 kb or greater around transcription start sites in the genomic sequence.

18. The medical decision support system of claim 16 or 17 wherein the neural network (306) comprises a graph neural network (210) arranged based on protein-protein interactions.

19. A method for the analysis of genomic sequence data, the method comprising:
obtaining (100) genomic sequence data of a patient;
translating (130) protein-coding gene sequences contained in the genomic sequence data into encoded protein sequences;
applying (135) a protein language model (302) to the encoded protein sequences, the applying resulting in first metric scores that assess functionality of corresponding proteins;
extracting (140) DNA sequences from the genomic sequence data that exert a regulatory effect on expression of the protein-coding genes;
applying (145) a regulatory DNA language model (304) to the extracted DNA sequences, the applying resulting in second metric scores that assess the expression of the corresponding protein-coding genes;
deriving (150) a diagnostic, prognostic, or predictive output with respect to a disease or medical condition by a machine-trained predictive model (306) in response to input of the first and second metric scores to the machine-trained predictive model; and
displaying (170) the diagnostic, prognostic, or predictive output.

20. The method of claim 19 wherein deriving (150) comprises deriving (150) by the machine-trained predictive model (306) comprises a graph neural network (210) where the graph represents protein-protein interactions.
